(19) **Europäisches Patentamt European Patent Office Office européen des brevets**

(11)  **EP 2 602 367 B1**

(12)  **EUROPEAN PATENT SPECIFICATION**

(45) Date of publication and mention
of the grant of the patent:
**13.05.2015  Bulletin 2015/20**

(51) Int Cl.:
***D04H 1/4291*** (2012.01)  ***D04H 1/407*** (2012.01)
***D01D 5/098*** (2006.01)

(21) Application number: **11192142.5**

(22) Date of filing: **06.12.2011**

(54) **PP copolymers for melt blown/pulp fibrous nonwoven structures with improved mechanical properties and lower hot air consumption**

PP-Copolymere für schmelzgeblasene/Faserstoffvliesstrukturen mit verbesserten mechanischen Eigenschaften und niedrigerem Warmluftverbrauch

Copolymères PP pour structures fibreuses de pâte/fabriquées par fusion-soufflage dotées de propriétés mécaniques améliorées et d'une consommation plus faible en air chaud

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**12.06.2013  Bulletin 2013/24**

(73) Proprietor: **Borealis AG**
**1220 Vienna (AT)**

(72) Inventors:
• **Fiebig, Joachim**
**4502 St. Marien (AT)**
• **Sars, Wilhelmus Henricus Adolf**
**5036 CA Tilburg (NL)**

• **Wimmer, Heinz**
**4114 Neuhaus (AT)**
• **Van Paridon, Henk**
**3271 Averbode (BE)**

(74) Representative: **Lux, Berthold**
**Maiwald Patentanwalts GmbH**
**Elisenhof**
**Elisenstraße 3**
**80335 München (DE)**

(56) References cited:
**WO-A1-2007/024447   WO-A2-2011/077277**
**WO-A2-2011/092092**

**Description**

The present invention is directed to a new composite material comprising melt blown fiber based on a propylene copolymer and a fibrous substrate material, a process for the preparation of such a composite material, articles made therefrom as well as to the use of the propylene copolymer for the preparation of such a composite material or article and the use of the propylene copolymer for reducing the hot air volume during a melt blowing process.

WO 2011/092092 describes a melt blown web based on melt blown fibers comprising a propylene copolymer and to articles made therefrom.

WO 2011/077277 describes a resilient coform nonwoven web that contains a matrix of meltblown fibers and an absorbent material.

WO 2007/024447 describes propylene-based nonwoven layers made by a meltblown process, and laminates incorporating such layers.

A melt blown web, being a non-woven structure consisting of melt blown fibers, is typically made in a one-step process in which high-velocity air blows a molten thermoplastic resin from an extruder die tip onto a conveyor or take-up screen to form fine fibered self-bonding web. Although many types of polymers can be employed for melt blown fibers and corresponding webs, polypropylene is one of the most commonly used polymers. Normally for the manufacture of melt blown webs and fibrous composite materials propylene homopolymers are used. However, such polymers and respective materials made therefrom typically suffer from limited mechanical strength. For example, the adhesion between a respective fibrous substrate material and a melt blown web used for laminating is often reduced such that a reduced tensile strength of the overall end product can be observed. One approach to overcome this problem is to combine melt blown webs with spunbonded webs, wherein the spunbonded web is acting as a support layer. Such combined melt blown/spunbonded webs can be produced directly in line (SMS) or can be laminated separately. However, the use of polypropylene melt blown webs is limited as the combination of melt blown webs and spunbonded webs are not sufficient for all applications and, furthermore, the poor mechanical properties of the melt blown web often causes problems during the converting processes.

In addition to polymer properties, the melt blown process parameters are crucial in production of melt blown and corresponding end products. During melt blown web and end product preparation, a significant cost is generated by hot air, which is used to attenuate the fibers of the web. Therefore, from melt blown process point of view, it is economical to have a polymer resin, which can be used to achieve same or better final melt blown web properties at lower air volumes.

Thus, the object of the present invention is to provide a composite material/article comprising the composite material with sufficient mechanical properties, in particular adhesion properties between the melt blown fibers and/or the melt blown web based thereon and a fibrous substrate material laminated with such melt blown fibers/web. A further object is that an economical manufacture of said composite material/article is achieved, i.e. the composite material/article with improved adhesion properties is obtained at rather low air volumes.

The finding is to provide a composite material/article comprising melt blown fiber and/or a melt blown web based on a propylene copolymer with a comonomer content not exceeding 5.5 wt.-% and having a melt flow rate $MFR_2$ (230 °C) measured according to ISO 1133 of at least 200 g/10min.

Thus, the present invention is directed to a composite material comprising

(a) melt blown fiber, wherein

(i) said melt blown fiber comprises at least 85 wt.-% of a propylene copolymer having a comonomer content of 0.5 wt.-% to 7.0 wt.-%, like 0.5 wt.-% to 5.5 wt.-%, the comonomer is ethylene and/or at least one $C_4$ to $C_{20}$ $\alpha$-olefin,
(ii) optionally said melt blown fiber and/or said propylene copolymer has/have a melt flow rate $MFR_2$ (230 °C) measured according to ISO 1133 of at least 200 g/10min, and

(b) at least one fibrous substrate material, wherein
the composite material comprises to 30 wt.-% of the melt blown fibre and 70 wt.-% to 99 wt.-% of the fibrous substrate material, based on the total weight of the composite material..

Surprisingly it has been found out that such a composite material/article comprising said composite material has much better mechanical properties, in particular adhesion properties, compared to known composite materials/articles comprising a propylene homopolymer. Furthermore, also the air volume required during the melt blowing process can be significantly reduced without losing the required properties of the melt-blown web.

According to another aspect of the present invention, an article comprising said composite material is provided, said article is selected from filtration media and/or absorbent products such as hygiene articles and/or tissue articles. It is preferred that said article has a maximum tensile strength a) in machine direction (MD) of at least 10N measured according to ISO 527-3 on rectangular specimens cut from the article having 50 mm width and 140 mm length at a temperature of +23 ± 2 °C and a testing speed of 100 mm/min and/or b) in cross direction (CD) of at least 10 N measured according to ISO 527-3 on rectangular specimens cut from the article having 50 mm width and 140 mm length at a temperature of +23 ± 2 °C and a testing speed of 100 mm/min.

According to a further aspect of the present invention, a process for the preparation of said composite material is provided, the process comprises the steps of

(a) providing a melt blown fiber prepared from the propylene copolymer,
(b) providing at least one fibrous substrate material,
(c) contacting a stream of the at least one fibrous substrate material of step (b) and a stream of the melt blown fiber of step (a) to form a composite material.

According to a still further aspect of the present invention, the use of a propylene copolymer for the preparation of said composite material or article is provided.

According to a further aspect of the present invention, the use of a propylene copolymer for reducing the hot air volume during a melt blowing process is provided, wherein the reduction is defined by Formula (I)

$$(AV\text{-}RPP) / (AV\text{-}HPP) \leq 0.9 \qquad (I)$$

wherein
(AV-RPP) is the hot air volume $[m^3/h]$ of the propylene copolymer, and (AV-HPP) is the hot air volume $[m^3/h]$ of a propylene homopolymer having a melt flow rate $MFR_2$ (230 °C) measured according to ISO 1133 of 1100 g/10min to 1300 g/10min and a melting temperature $(T_m)$ measured according to ISO 11357-3 of 155°C to 160 °C.

When in the following reference is made to preferred embodiments or technical details of the composite material, it is to be understood that these preferred embodiments or technical details also refer to the inventive article, the inventive process as well as the inventive uses. If, for example, it is set out that the inventive composite material preferably comprises a propylene copolymer and/or melt blown fiber having a melting temperature of at least 130 °C, also the composite material provided in the inventive article, the inventive process as well as the inventive uses preferably comprise a propylene copolymer and/or melt blown fiber having a melting temperature of at least 130°C.

According to one preferred embodiment of the present invention, the propylene copolymer and/or melt blown fiber has/have a melting temperature of at least 130°C.

According to another preferred embodiment of the present invention, the melt blown fiber and/or propylene copolymer has/have a melt flow rate $MFR_2$ (230°C) measured according to ISO 1133 in the range of 200 g/10min and 3000 g/10min.

According to yet another preferred embodiment of the present invention, the propylene copolymer has <2,1> regiodefects of not more than 0.4 mol.-% determined by $^{13}C$-spectroscopy.

According to one preferred embodiment of the present invention, the pentad isotacticity of the propylene copolymer is higher than 95 mol.-% determined with $^{13}C$ NMR spectroscopy.

According to another preferred embodiment of the present invention, the propylene copolymer has been visbroken with a visbreaking ratio [final $MFR_2$ (230°C) / initial $MFR_2$ (230°C)] of 5 to 100, wherein "initial $MFR_2$ (230°C)" is the $MFR_2$ (230°C) of the propylene copolymer before visbreaking and "final $MFR_2$ (230 °C)" is the $MFR_2$ (230 °C) of the propylene copolymer after visbreaking.

According to yet another preferred embodiment of the present invention, the comonomer of the propylene copolymer is ethylene.

According to one preferred embodiment of the present invention, the fibrous substrate material is based on a material selected from the group consisting of wood pulp, rayon, lyocell, cotton, wool, silk, jute, linen, hemp, flax, polyester, nylon, polyethylene, polyethylene terephthalate, polycarbonate, polyacrylate, salts of polyacrylate and mixtures thereof.

According to another preferred aspect, the composite material comprises from 1 wt.-% to 60 wt.-% of the melt blown fiber and from 40 wt.-% to 99 wt.-% of the at least one fibrous substrate material, more preferably comprises from 10 wt.-% to 50 wt.-% of the melt blown fiber and from 50 wt.-% to 90 wt.-% of the at least one fibrous substrate material, yet more preferably from 20 wt.-% to 40 wt.-% of the melt blown fiber and from 60 wt.-% to 80 wt.-% of the at least one fibrous substrate material

According to yet another preferred embodiment of the present invention, the melt blown fiber are in form of a melt blown web.

According to another preferred embodiment of the present invention, the melt blown web has a maximum tensile strength a) in machine direction (MD) of at least 10 N measured according to ISO 527-3 on rectangular specimens cut from the web having 50 mm width and 140 mm length at a temperature of +23 ± 2 °C and a testing speed of 100 mm/min and/or b) in cross direction (CD) of at least 5 N measured according to ISO 527-3 on rectangular specimens cut from the web having 50 mm width and 140 mm length at a temperature of +23 ± 2 °C and a testing speed of 100 mm/min.

In the following the composite material according to the instant invention is defined in more detail.

One essential requirement is that the fiber being part of the inventive composite material is a melt blown fiber. Melt blown fibers differ essentially from other fibers, in particular from those produced by spunbond technique. In the melt blowing process a gas stream of high velocity impinges on the molten polymer stream as the polymer extrudes from small capillary in the melt blowing die and rapidly attenuates the polymer filament from about 500 $\mu$m diameter at the capillary down to diameters below 5.0 $\mu$m, like down to diameters below 3.0 $\mu$m. This corresponds to a reduction of 500 times in fiber diameter and 2,500,000 times in cross sectional area. The process occurs in about 200 microseconds over a distance of a few centimeters. This amounts to 6 times more surface area and 36 times more fiber in a melt blown web of 1 to 3 $\mu$m compared to an equivalent fiber web produced by carded or spunbond technique. Thus, the principal advantage of the melt blown process is that one can make very fine fibers and very lightweight melt blown webs with excellent uniformity. The result is a soft melt blown web with excellent barrier properties, meaning effective filtration characteristics and resistance to penetration by aqueous liquids. In other words the process features "melt blown" distinguishes such produced fibers from fibers produced by different technology. More precisely "melt blown fibers" are very thin having diameters not accomplished with other fiber processes. Furthermore, webs made out of such melt blown fibers are softer and have lower weight compared to webs of the same thickness but produced by other technologies, like the spunbond process.

Accordingly, the melt blown fiber according to the present invention preferably has an (average) diameter measured of not more than 5 $\mu$m, like below 5 $\mu$m, more preferably of not more than 3$\mu$m. It is in particular appreciated that the (average) diameter of the melt blown fiber is in the range of 0.1 $\mu$m to 5 $\mu$m, more preferably in the range of 0.5 $\mu$m to 4.9 $\mu$m, yet more preferably in the range of 0.5 $\mu$m to 3 $\mu$m, like 1 $\mu$m to 3 $\mu$m.

One specific requirement of the present invention is that the melt blown fiber comprises a propylene copolymer. Preferably the melt blown fiber comprises at least 85 wt.-%, more preferably at least 90 wt.-%, like at least 95 wt.-% of the propylene copolymer, based on the total weight of the melt blown fiber. Accordingly, it is in particular appreciated that the melt blown fiber may comprise in addition to the propylene copolymer typical additives, like antioxidants stabilizers, fillers, colorants, nucleating agents and mold release agents. Primary and secondary antioxidants include, for example, hindered phenols, hindered amines, and phosphates. Nucleating agents include, for example, sodium benzoate, sorbitol derivatives like bis-(3,4-dimethylbenzylidene)sorbitol and nonitol derivatives like 1,2,3-trideoxy-4,6:5,7-bis-O[(4-propylphenyl)methylene]-nonitol. Other additives such as dispersing agents like glycerol monostearate can also be included. Slip agents include, for example, oleamide and erucamide. Catalyst deactivators are also commonly used, for example, calcium stearate, hydrotalcite, and calcium oxide, and/or other acid neutralizers known in the art. The amount of such additives however shall preferably not exceed 10 wt.-%, more preferably not more than 5 wt.-%, based on the total weight of the melt blown fiber and/or web comprising the melt blown fiber. Accordingly, in a specific embodiment the melt blown fiber and/or the web comprising the melt blown fiber may contain additives, in particular those as stated in this paragraph, but no other polymers. Thus, it is preferred that the propylene copolymer is the only polymer within the melt blown fiber and/or web comprising the melt blown fiber.

In the following the propylene copolymer is described in more detail.

A "comonomer" according to this invention is a polymerizable unit different to propylene. Accordingly, the propylene copolymer according to this invention has a comonomer content of 0.5 wt.-% to 7.0 wt.-%, like 0.5 wt.-% to 5.5 wt.-%, preferably of 0.5 wt.-% to 5 wt.-%, more preferably of 0.5 wt.-% to 4.5 wt.-% and most preferably of 1 to 4 wt.-%, based on the total weight of the propylene copolymer. For example, the propylene copolymer has a comonomer content of 1 wt.-% to 3.5 wt.-%, based on the total weight of the propylene copolymer.

The remaining part constitutes units derivable from propylene. Accordingly, the propylene content in the propylene copolymer is preferably at least 94 wt.-%, more preferably at least 93.0 wt.-%, like at least 94.5 wt.-%, yet more preferably in the range of 93.0 wt.-% to 99.5 wt.-%, like 94.5 wt.-% to 99.5 wt.-%, still more preferably 95 wt.-% to 99.5 wt.-%, still yet more preferably 95.5 wt.-% to 99.5 wt.-% and most preferably 96 wt.-% to 99 wt.-%, based on the total weight of the propylene copolymer. For example, the propylene copolymer has a propylene content of 96.5 wt.-% to 99 wt.-%, based on the total weight of the propylene copolymer. -

In one preferred embodiment of the present invention, the propylene copolymer has a propylene content of 96.5 wt.-% to 99 wt.-% and a comonomer content of 1 wt.-% to 3.5 wt.-%, based on the total weight of the propylene copolymer.

The comonomer of the propylene copolymer is ethylene and/or at least one $C_4$ to $C_{20}$ $\alpha$-olefin, more preferably the comonomer of the propylene copolymer is selected from the group consisting of ethylene, $C_4$ $\alpha$-olefin, $C_5$ $\alpha$-olefin, $C_6$ $\alpha$-olefin, $C_7$, $\alpha$-olefin, $C_8$ $\alpha$-olefin, $C_9$ $\alpha$-olefin and $C_{10}$ $\alpha$-olefin still more preferably the comonomer of the propylene copolymer is selected from the group consisting of ethylene, 1-butene, 1-pentene, 1-hexene, 1-heptene, 1-octene, 1-nonene and 1-decene, wherein ethylene, 1-butene and 1-hexene are preferred.

The propylene copolymer may contain more than one type of comonomer. Thus, the propylene copolymer of the present invention may contain one, two or three different comonomers. However it is preferred that the propylene copolymer contains only one type of comonomer. Preferably the propylene copolymer comprises - apart from propylene - only ethylene, 1-butene, or 1-hexene.

In one preferred embodiment of the present invention, the comonomer of the propylene copolymer is only ethylene.

Thus, the propylene copolymer is in a preferred embodiment a propylene copolymer of propylene and ethylene only, wherein the ethylene content is in the range of 0.5 wt.-% to 5.5 wt.-%, preferably in the range of 0.5 wt.-% to 4.5 wt.%, more preferably in the range of 1 wt.-% to 4 wt.-%, based on the total weight of the propylene copolymer. For example, the propylene copolymer consist of propylene and ethylene only, wherein the ethylene content is in the range of 1 wt.-% to 3.5 wt.-%, based on the total weight of the propylene copolymer. Furthermore, it is appreciated that the xylene soluble content of the propylene copolymer and/or of the melt blown fibers, preferably of the propylene copolymer, is rather low. Accordingly, the propylene copolymer and/or the melt blown fiber, preferably the propylene copolymer, has preferably a xylene cold soluble fraction (XCS) measured according to ISO 6427 (23 °C) of not more than 12 wt.-%, more preferably of not more than 10 wt.-%, yet more preferably of not more than 9.5 wt.-%, like not more than 9 wt.-%. Thus, a preferred range is 1 wt.-% to 12 wt.-%, more preferred 1.5 wt.-% to 10 wt.-%, still more preferred 2 wt.-% to 9 wt.-%.

A further characteristic of the propylene copolymer is the low amount of missinsertions of propylene within the polymer chain. Accordingly, the propylene copolymer is featured by a low amount of <2,1> regiodefects, i.e. of not more than 0.4 mol.-%, more preferably of not more than 0.2 mol.-%, like of not more than 0.1 mol.-%, determined by $^{13}$C-NMR spectroscopy.

Additionally or alternatively, it is appreciated that the propylene copolymer and/or the melt blown fiber has/have a heat of fusion (Hm) of at least 80 J/g, more preferably of at least 90 J/g, still more preferably in the range of 85 J/g to 110 J/g, yet more preferably in the range of 90 J/g to 105 J/g as determined by DSC.

Furthermore, it is preferred that the propylene copolymer and/or the melt blown fiber has/have a crystallinity of at least of 35 %, more preferably in the range of 40 % to 55 %.

Preferably, the random propylene copolymer is isotactic. Accordingly, it is appreciated that the propylene copolymer has a rather high pentad concentration, i.e. higher than 95 mol-%, more preferably higher than 97 mol-%, still more preferably higher than 98 mol-% as determined with $^{13}$C-NMR spectroscopy.

Furthermore, it is appreciated that the comonomers within the propylene copolymer are randomly distributed. The randomness indicates the amount of isolated comonomer units, i.e. those which have no other comonomer units in the neighbour, compared to the total amount of comonomers in the polymer chain. In a preferred embodiment of the present invention, the randomness of the propylene copolymer is at least 30 %, more preferably at least 50 %, even more preferably at least 60 %, and still more preferably at least 65 %.

As stated above the propylene copolymer is preferably the only polymer component within the melt blown fiber. Accordingly the term "propylene copolymer" according to this invention does not encompass heterophasic systems comprising a polypropylene and dispersed therein an elastomeric component. In fact the propylene copolymer according to this invention shall preferably not be understood as a mixture of two different polymers being not miscible. The term being "not miscible" indicates polymer mixtures wherein the different polymers due to their different nature form distinguishable phases visible by high resolution microscopy, like electron microscopy or scanning force microscopy. However, this does not exclude the option that the propylene copolymer is a so called bimodal or multimodal polymer. Different to non miscible polymers, bimodal or multimodal polymers comprise fractions which differ in their molecular weight distribution and/or their comonomer content distribution but nevertheless are miscible in the meaning of the invention.

Thus expression "multimodal" or "bimodal" used herein refers to the modality of the polymer, i.e. the form of its comonomer content distribution curve, which is the graph of the comonomer content as a function of elution temperature of the temperature rising elution fractionation (TREF) method. The temperature rising elution fractionation (TREF) technique fractionates propylene polymers according to the longest crystallizable sequences in the chain, which increases almost linearly with the elution temperature (P. Ville et al., Polymer 42 (2001) 1953-1967). Hence, the higher the maximum temperature the longer are the isotactic sequences. Furthermore, the temperature rising elution fraction (TREF) technique does not strictly fractionate polypropylene according to tacticity but according to the longest crystallizable sequences in the chain. Hence, the solubility of the propylene copolymer chain is influenced by the concentration and distribution of sterical defects. Insofar the temperature rising elution fraction (TREF) technique is an appropriate method to characterize the propylene copolymer in view of its comonomer distribution further.

As will be explained below the propylene copolymer can be produced by blending different polymer types, i.e. of comonomer content. However, it is preferred that the polymer components of the propylene copolymer are produced in a sequential step process, using reactors in serial configuration and operating at different reaction conditions. As a consequence, each fraction prepared in a specific reactor will have its own comonomer content distribution.

When the distribution curves from these fractions are superimposed to obtain the comonomer content distribution curve of the final polymer, these curves may show two or more maxima or at least be distinctly broadened when compared with curves for the individual fractions. Such a polymer, produced in two or more serial steps, is called bimodal or multimodal, depending on the number of steps.

Accordingly, the propylene copolymer according to this invention may be bimodal or multimodal but not a mixture of non-miscible polymers (polymer fractions). Thus, in one embodiment of the present invention, the propylene copolymer is multimodal, like bimodal, in view of the comonomer content. In another preferred embodiment the propylene copolymer is unimodal in view of the comonomer distribution.

Furthermore, it is appreciated that there is a dependency between the melting temperature and the comonomer, in particular ethylene, content within the propylene copolymer. In this regard, it is known that with increase of the comonomer content, in particular with increase of ethylene, the melting temperature decreases. In one preferred embodiment of the present invention, the melting temperature and the comonomer content, i.e. ethylene content, must comply a specific relationship.

Preferably, the propylene copolymer and/or the melt blown fiber according to the instant invention fulfills the equation (1), more preferably the equation (1a), yet more preferably the equation (Ib),

$$\frac{Tm\ [°C]}{[°C]} \geq 160 - \frac{C2\ [wt\%]\ \times 5.25}{[wt\%]} \quad (1)$$

$$\frac{Tm\ [°C]}{[°C]} \geq 161 - \frac{C2\ [wt\%]\ \times 5.25}{[wt\%]} \quad (1a)$$

$$\frac{Tm\ [°C]}{[°C]} \geq 162 - \frac{C2\ [wt\%]\ \times 5.25}{[wt\%]} \quad (1b)$$

wherein

Tm [°C]    is the melting temperature [given in °C] of the melt blown fiber and/or of propylene copolymer, preferably of the propylene copolymer, measured according to ISO 11357-3,

C2 [wt.-%]    is the amount [given in weight percentage] of comonomer, preferably of ethylene, within the melt blown fiber and/or the propylene copolymer, preferably within the propylene copolymer, determined with Fourier transform infrared spectroscopy (FTIR).

Furthermore, it is appreciated that the propylene copolymer and/or the melt blown fiber according to this invention has/have a melting temperature Tm measured according to ISO 11357-3 of least 130°C, preferably at least 135°C, more preferably at least 137°C, like of at least 140°C. Accordingly the melting temperature ranges preferably from 130°C to 157°C and more preferably from 135°C to 155°C.

A further mandatory requirement of the propylene copolymer and/or of the melt blown fiber is its rather high melt flow rate, which differ(s) from other polymers and/or fibers obtained by spunbond technique. The melt flow rate mainly depends on the average molecular weight. This is due to the fact that long molecules render the material a lower flow tendency than short molecules. An increase in molecular weight means a decrease in the MFR-value. The melt flow rate (MFR) is measured in g/10 min of the polymer discharged through a defined die under specified temperature and pressure conditions and the measure of viscosity of the polymer which, in turn, for each type of polymer is mainly influenced by its molecular weight but also by its degree of branching. The melt flow rate measured under a load of 2.16 kg at 230°C (ISO 1133) is denoted as $MFR_2$ (230°C).

Accordingly, it is preferred that in the present invention the propylene copolymer and/or the melt blown fiber has/have an $MFR_2$ (230°C) of at least 200 g/10min more preferably of at least 400 g/10min, still more preferably in the range 200 g/10min to 3000 g/10 min, yet more preferably in the range of 400 to 2000 g/10 min.

In one preferred embodiment of the present invention, the propylene copolymer and/or the melt blown fiber has/have an $MFR_2$ (230 °C) in the range of 600 g/10min to 1800 g/10 min and more preferably in the range of 800 g/10min to 1600 g/10 min. For example, the propylene copolymer and/or the melt blown fiber has/have an $MFR_2$ (230 °C) in the range 1000 g/10min to 1400 g/10 min, like 1100 g/10min to 1300 g/10 min.

A propylene copolymer with such high melt flow rate is preferably obtained by vis-breaking. Accordingly, it is appreciated that the propylene copolymer according to this invention is obtained by visbreaking a propylene polymer with the same properties as indicated in the instant invention but with a lower melt flow rate ($MFR_z$).

Thus, it is preferred that the propylene copolymer before visbreaking has an $MFR_2$ (230 °C) of not more than 150 g/10min, more preferably in the range of 15 g/10min to 120 g/10min, yet more preferably in the range of 20 g/10min to 100 g/10min.

Preferably, the initially used propylene copolymer is chosen in such a manner that the visbreaking ratio [final MFR$_2$ (230 °C) / initial MFR$_2$ (230 °C)] is 5 to 100, wherein "initial MFR$_2$ (230 °C)" is the MFR$_2$ (230 °C) of the propylene copolymer before visbreaking and "final MFR$_2$ (230 °C)" is the MFR$_2$ (230 °C) of the propylene copolymer after visbreaking.

In one preferred embodiment of the present invention, the propylene copolymer has been visbroken with a visbreaking ratio [final MFR$_2$ (230 °C) / initial MFR$_2$ (230 °C)] of 20 to 100, more preferably 20 to 80, even more preferably 30 to 80 and most preferably 40 to 80, wherein "initial MFR$_2$ (230 °C)" is the MFR$_2$ (230 °C) of the propylene copolymer before visbreaking and "final MFR$_2$ (230 °C)" is the MFR$_2$ (230 °C) of the propylene copolymer after visbreaking.

Additionally or alternatively, the propylene copolymer is featured by a rather narrow molecular weight distribution as the polymer has been preferably visbroken. Visbreaking of polymers not only increases the melt flow rate but additionally narrows the molecular weight distribution. Accordingly, it is appreciated that the molecular weight distribution ($M_w/M_n$) of the propylene copolymer and/or of the melt blown fiber is in the range of 2 to 6, more preferably in the range of 2.5 to 5.5.

Furthermore, the propylene copolymer and/or the melt blown fiber made from the propylene copolymer is featured by a specific tensile modulus as measured according to ISO 527-3 using injection molded specimen as described in EN ISO 1873-2 (dog bone shape, 4 mm thickness). Thus, it is preferred that the propylene copolymer and/or the melt blown fiber has a tensile modulus of at least 500 MPa, more preferably of at least 700 MPa and most preferably in the range of 500 MPa to 1400 MPa. For example, the propylene copolymer and/or the melt blown fiber has a tensile modulus in the range of 700 MPa to 1200 MPa and most preferably in the range of 800 MPa to 1000 MPa.

Furthermore, the propylene copolymer and/or the melt blown fiber made from the propylene copolymer is/are featured by a specific relationship of tensile modulus to comonomer content within the propylene copolymer and/or fiber. Thus, it is preferred that the propylene copolymer and/or the melt blown fiber fulfill(s) the equation (2), more preferably the equation (2a),

$$\frac{TM\ [MPa]}{[MPa]} \geq 1200 + \frac{C2\ [wt\%] \times 145}{[wt\%]} \quad (2)$$

$$\frac{TM\ [MPa]}{[MPa]} \geq 1250 + \frac{C2\ [wt\%] \times 145}{[wt\%]} \quad (2a)$$

wherein

TM [MPa]    is the tensile modulus [given in MPa] of the melt blown fiber and/or of the propylene copolymer, preferably within the propylene copolymer, measured according to ISO 527-3 using injection molded specimen as described in EN ISO 1873-2 (dog bone shape, 4 mm thickness),

C2 [wt.-%]    is the amount [given in weight percentage] of comonomer, preferably of ethylene, within the melt blown fiber and/or within the propylene copolymer, preferably within said propylene copolymer, determined with Fourier transform infrared spectroscopy (FTIR).

In one preferred embodiment of the present invention, the melt blown fiber are provided in the form of a melt blown web.

Accordingly, the melt blown web is preferably a nonwoven web. The term "nonwoven web" in the meaning of the present invention refers to a web comprising individual melt blown fiber that have been formed into a web and bonded together by any means such as by chemical, mechanical, heat and/or solvent treatment, but not by weaving or knitting.

Preferably, the melt blown web according to the instant invention has a maximum tensile strength

(a) in the machine direction (MD) of at least 10 N measured according to ISO 527-3 on rectangular specimens cut from the web having 50 mm width and 140 mm length at a temperature of +23 ± 2 °C and a testing speed of 100 mm/min and/or

(b) in the cross direction (CD) of at least 5 N measured according to ISO 527-3 on rectangular specimens cut from the web having 50 mm width and 140 mm length at a temperature of +23 ± 2 °C and a testing speed of 100 mm/min.

For example, the melt blown web according to the instant invention has a maximum tensile strength in machine direction (MD) of between 10 N and 35 N and preferably between 15 N and 30 N, measured according to ISO 527-3 on rectangular specimens cut from the web having 50 mm width and 140 mm length at a temperature of +23 $\pm$ 2 °C and a testing speed of 100 mm/min.

Additionally or alternatively, the melt blown web according to the instant invention has a maximum tensile strength in cross direction (CD) of between 5 N and 30 N and preferably between 5 N and 20 N, measured according to ISO 527-3 on rectangular specimens cut from the web having 50 mm width and 140 mm length at a temperature of +23 $\pm$ 2 °C and a testing speed of 100 mm/min.

The basis weight of the melt blown web being part of the composite material depends very much on the end use, however it is preferred that the melt blown web has a basis weight of at least 15 g/m$^2$.

Preferably, the melt blown web of the present invention has a basis weight of at least 20 g/m$^2$, more preferably at least 25 g/m$^2$, even more preferably at least 30 g/m$^2$ and most preferably of at least 35 g/m$^2$. Accordingly, the melt blown web of the present invention has a basis weight of between 20 g/m$^2$ and 150 g/m$^2$, more preferably between 25 g/m$^2$ and 125 g/m$^2$, even more preferably between 30 g/m$^2$ and 100 g/m$^2$ and most preferably between 35 g/m$^2$ and 75 g/m$^2$. For example, the melt blown web of the present invention has a basis weight of between 35 g/m$^2$ and 65 g/m$^2$ and more preferably between 40 g/m$^2$ and 60 g/m$^2$.

In the following the preparation of the propylene copolymer is described in more detail.

The propylene copolymer as defined in the instant invention may be prepared by polymerizing, in a slurry reactor, for example a loop reactor, propylene optionally together with ethylene and/or at least another $C_4$ to $C_{20}$ $\alpha$-olefin (comonomers), in the presence of a polymerization catalyst to produce a part of the propylene copolymer. This part is then transferred to a subsequent gas phase reactor, whereupon in the gas phase reactor propylene is reacted in the presence of suitably selected other ethylene and/or at least one $C_4$ to $C_{20}$ $\alpha$-olefin(s) (comonomers) in order to produce a further part in the presence of the reaction product of the first step. This reaction sequence provides a reactor blend of parts (i) and (ii) constituting the propylene copolymer. It is of course possible by the present invention that the first reaction is carried out in a gas phase reactor while the second polymerization reaction is carried out in a slurry reactor, for example a loop reactor. It is furthermore also possible to reverse the order of producing parts (i) and (ii), which has been described above in the order of first producing part (i) and then producing part (ii). The above-discussed process, comprising at least two polymerization steps, is advantageous in view of the fact that it provides easily controllable reaction steps enabling the preparation of a desired reactor blend. The polymerization steps may be adjusted, for example by appropriately selecting monomer feed, comonomer feed, hydrogen feed, temperature and pressure in order to suitably adjust the properties of the polymerization products obtained. It is in particular possible to obtain a multimodality, preferably the bimodality, of the propylene copolymer, with respect to the comonomer, like ethylene, distribution as well as with respect to the molecular weights and MFR$_2$ (230 °C) values during said multistage polymerization procedures.

Such a process can be carried out using any suitable catalyst for the preparation of the propylene copolymer. Preferably, the process as discussed above is carried out using a Ziegler-Natta catalyst, in particular a high yield Ziegler-Natta catalyst (so-called fourth and fifth generation type to differentiate from low yield, so called second generation Ziegler-Natta catalysts). A suitable Ziegler-Natta catalyst to be employed in accordance with the present invention comprises a catalyst component, a co-catalyst component and at least one electron donor (internal and/or external electron donor, preferably at least one external donor). Preferably, the catalyst component is a Ti-Mg-based catalyst component and typically the co-catalyst is an Al-alkyl based compound. Suitable catalysts are in particular disclosed in US 5,234,879, WO 92/19653, WO 92/19658 and WO 99/33843.

Preferred external donors are the known silane-based donors, such as dicyclopentyl dimethoxy silane or cyclohexyl methyldimethoxy silane.

One embodiment of a process as discussed above is a loop-gas phase process, such as developed by Borealis, known as Borstar® technology, described for example in EP 0 887 379 A1 and WO 92/12182.

With respect to the above-mentioned preferred slurry-gas phase process, the following general information can be provided with respect to the process conditions.

Temperature of from 40 °C to 110 °C, preferably between 60 °C and 100 °C, in particular between 80 °C and 90 °C, with a pressure in the range of from 20 bar to 80 bar, preferably 30 bar to 60 bar, with the option of adding hydrogen in order to control the molecular weight. The reaction product of the slurry polymerization, which preferably is carried out in a loop reactor, is then transferred to the subsequent gas phase reactor, wherein the temperature preferably is within the range of from 50 °C to 130 °C, more preferably 80 °C to 100 °C, at a pressure in the range of from 5 bar to 50 bar, preferably 15 bar to 35 bar, again with the option of adding hydrogen in order to control the molecular weight.

The residence time can vary in the reactor zones identified above. In one preferred embodiment of the present invention, the residence time in the slurry reaction, for example the loop reactor, is in the range of from 0.5 hours to 5 hours, for example 0.5 hours to 2 hours, while the residence time in the gas phase reactor generally will be from 1 hours to 8 hours.

The properties of the propylene copolymer produced with the above-outlined process may be adjusted and controlled with the process conditions as known to the skilled person, for example by one or more of the following process parameters: temperature, hydrogen feed, comonomer feed, propylene feed, catalyst, type and amount of external donor, split between two or more components of a multimodal polymer.

In case the propylene copolymer is subjected to a visbreaking step, the visbreaking may be carried out in any known manner, like by using a peroxide visbreaking agent. Typical visbreaking agents are 2,5-dimethyl-2,5-bis(tert.butyl-peroxy)hexane (DHBP) (for instance sold under the tradenames Luperox 101 and Trigonox 101), 2,5-dimethyl-2,5-bis(tert.butyl-peroxy)hexyne-3 (DYBP) (for instance sold under the tradenames Luperox 130 and Trigonox 145), dicumyl-peroxide (DCUP) (for instance sold under the tradenames Luperox DC and Perkadox BC), di-tert.butyl-peroxide (DTBP) (for instance sold under the tradenames Trigonox B and Luperox Di), tert.butyl-cumyl-peroxide (BCUP) (for instance sold under the tradenames Trigonox T and Luperox 801) and bis (tert.butylperoxyisopropyl)benzene (DIPP) (for instance sold under the tradenames Perkadox 14S and Luperox DC). Suitable amounts of peroxide to be employed in accordance with the present invention are in principle known to the skilled person and can easily be calculated on the basis of the amount of propylene copolymer to be subjected to visbreaking, the $MFR_2$ (230 °C) value of the propylene copolymer to be subjected to visbreaking and the desired target $MFR_2$ (230 °C) of the product to be obtained. Accordingly, typical amounts of peroxide visbreaking agent are from 0.005 wt.-% to 0.5 wt.-%, more preferably from 0.01 wt.-% to 0.2 wt.-%, based on the total amount of propylene copolymer employed.

Typically, visbreaking in accordance with the present invention is carried out in an extruder, so that under the suitable conditions, an increase of melt flow rate is obtained. During visbreaking, higher molar mass chains of the starting product are broken statistically more frequently than lower molar mass molecules, resulting as indicated above in an overall decrease of the average molecular weight and an increase in melt flow rate.

The thus obtained propylene polymer is used in pellet or granule form for the preparation of the inventive composite material.

One further requirement of the instant invention is that the composite material further comprises at least one fibrous substrate material.

Thus, in the following the at least one fibrous substrate material being part of the instant composite material is described in more detail.

It is appreciated that the term "fibrous substrate material" in the meaning of the present application refers to an elongated particulate material having a length typically exceeding its width. In this context, a fibrous substrate material refers to a material having a length of not more than 10 cm, more preferably of not more than 7 cm, yet more preferably in the range of 0.1 mm to 7 cm, still more preferably in the range of 0.1 to 50 mm, still yet more preferably in the range of 0.5 to 10 mm. Additionally or alternatively, such fibrous substrate material has a width (diameter) of not more than 80 $\mu$m, more preferably of not more than 50 $\mu$m, more preferably in the range of 1 to 40 $\mu$m. Accordingly, the fibrous substrate material preferably has a length to width ratio of at least 10, more preferably of at least 30, still more preferably in the range of 30 to 100, yet more preferably in the range of 40 to 80, like in the range of 50 to 70. The fibrous substrate material may be a monocomponent or multicomponent material, for example a bicomponent fibrous material.

For example, the fibrous substrate material may be based on a material selected from the group consisting of wood pulp, rayon, lyocell, cotton, wool, silk, jute, linen, hemp, flax, polyester, nylon, polyethylene, polyethylene terephthalate, polycarbonate and mixtures thereof.

Preferably, at least one fibrous substrate material is a cellulosic fiber. The term "cellulosic fiber" in the meaning of the present invention refers to a fiber based on cellulose, wherein the cellulose may be derived from any source, either natural or manufactured. The cellulosing fiber preferably comprises cellulose in an amount of at least 75 wt.-%, more preferably 80 wt.-%, even more preferably 85 wt.-% and most preferably at least 90 wt.-%, based on the total weight of the cellulosic fiber.

Preferably, the fibrous substrate material being a cellulosic fiber is based on a material selected from the group consisting of wood pulp, rayon, lyocell, cotton, wool, jute, linen, hemp, flax and mixtures thereof.

In one preferred embodiment of the present invention, the at least one fibrous substrate material being a cellulosic fiber is preferably a wood pulp fiber. Such cellulosic fiber may be obtained from various sources and qualities by processing and refining wood into fibres. Such processing and refining steps may include for example combinations of grinding, thermal and/or chemical treatment steps. The source of such cellulosic fiber is not critical for the present invention, i.e. the cellulosic fiber may be derived from softwood as well as hardwood trees.

Suitable wood pulp fiber may be obtained from a mechanical pulp, such as ground wood, thermo mechanical pulp (TMP) or chemothermomechanical pulp (CTMP), as well as chemical pulp, such as kraft pulp or sulphate pulp, or recycled pulp.

In this regard, "mechanical pulp" is typically prepared by milling chips of pulpwood into fibre components by using mechanical energy. "Ground wood pulp" is generally derived from softwood trees such as spruce, pine, fir, larch and hemlock, but also some hardwoods such as eucalyptus. Ground wood pulp is typically produced by grinding wood into relatively short fibres by using stones. "Thermomechanical pulp" is derived from a thermo-mechanical process wherein wood chips or saw dust is softened by steam before entering a pressurized refiner. "Chemothermomechanical pulp" is derived by treating wood chips with chemical agents such as sodium sulfite and steam and subsequent mechanical treatment. "Chemical pulp" is derived by treating wood chips or saw dust with chemical agents to release the cellulosic fibers by removing binding agents such as lignin resins and gums. Sulphate or Kraft are two types of chemical pulps wherein Kraft is the predominant pulping process in chemical pulp production. "Recycled pulp" is derived from recycled paper and paperboard or wastepaper. Recycled pulp may, in addition to the cellulosic fibers, comprise further materials such as fillers and adhesives used to facilitate the original papermaking.

In one preferred embodiment of the present invention, the at least one fibrous substrate material being a cellulosic fiber is derived from chemical pulp and/or recycled pulp.

In another preferred embodiment of the present invention, the at least one fibrous substrate material comprises a mixture of at least two fibrous substrate materials, wherein at least one fibrous substrate material is a cellulosic fiber as defined above. Preferably, the fibrous substrate material comprises a mixture of two fibrous substrate materials, i.e. a cellulosic fiber as defined above and a further type of a fibrous substrate material. More preferably, the fibrous substrate material comprises wood pulp fiber as cellulosic fiber and a further type of a fibrous substrate material. For example, the further type of fibrous substrate material is based on a material selected from the group consisting of rayon, lyocell, cotton, wool, silk, jute, linen, hemp, flax, polyester, nylon, polyethylene, polyethylene terephthalate, polyacrylate, salts of polyacrylate and polycarbonate. Preferably, the further type of fibrous substrate material is based on a material selected from the group consisting of polyester, nylon, polyethylene, polyethylene terephthalate, polyacrylate, salts of polyacrylate and polycarbonate.

In one preferred embodiment of the present invention, the further type of fibrous substrate material constitutes less than 50 wt.-%, based on the total weight of the fibrous substrate material, preferably less than 40 wt.-% and more preferably less than 30 wt.-%. Accordingly, it is appreciated that the cellulosic fiber constitutes at least 50 wt.-%, based on the total weight of the fibrous substrate material, preferably at least 60 wt.-% and more preferably at least 70 wt.-%.

Preferably, the fibrous substrate material comprises between 50 wt.-% and 99 wt.-% of the cellulosic fiber and between 1 wt.-% and 50 wt.-% of the further type of fibrous substrate material, based on the total weight of the fibrous substrate material. More preferably, the fibrous substrate material comprises between 60 wt.-% and 99 wt.-% of the cellulosic fiber and between 1 wt.-% and 40 wt.-% of the further type of fibrous substrate material, based on the total weight of the fibrous substrate material. Most preferably, the fibrous substrate material comprises between 70 wt.-% and 99 wt.-% of the cellulosic fiber and between 1 wt.-% and 30 wt.-% of the further type of fibrous substrate material, based on the total weight of the fibrous substrate material.

For example, the fibrous substrate material comprises between 90 wt.-% and 99 wt.-% of wood pulp fiber and between 1 wt.-% and 10 wt.-% of a further type of a fibrous substrate material, based on the total weight of the fibrous substrate material. Preferably, the further type of fibrous substrate material is based on polyethylene terephthalate (PET).

Alternatively, the fibrous substrate material comprises between 65 wt.-% and 75 wt.-% of wood pulp fiber and between 25 wt.-% and 35 wt.-% of a further type of a fibrous substrate material, based on the total weight of the fibrous substrate material. Preferably, the further type of fibrous substrate material is based on polyethylene terephthalate (PET).

In another preferred embodiment of the present invention, the fibrous substrate material comprises between 75 wt.-% and 99 wt.-% of wood pulp fiber and between 1 wt.-% and 25 wt.-% of a further type of a fibrous substrate material, based on the total weight of the fibrous substrate material. Preferably, the further type of fibrous substrate material is based on polyacrylate and/or salt of polyacrylate. For example, the salt of polyacrylate is sodium acrylate.

It is preferred that the composite material comprises the melt blown fibre and/or melt blown web based on the melt blown fibers in an amount of at least 1 wt.-%, preferably of at least 3 wt.-%, more preferably of at least 5 wt.-% and most preferably at least 7 wt.-%, based on the total weight of the composite material. Accordingly, it is appreciated that the composite material comprises the fibrous substrate material in an amount of less than 99 wt.-%, preferably of less than 97 wt.-%, more preferably of less than 95 wt.-% and most preferably of less than 93 wt.-%, based on the total weight of the composite material.

In one preferred embodiment of the present invention, the composite material comprises from 1 wt.-% to 30 wt.-% of the melt blown fibre and/or melt blown web based on the melt blown fiber and from 70 wt.-% to 99 wt.-% of the fibrous substrate material, based on the total weight of the composite material. Preferably, the composite material comprises from 3 wt.-% to 30 wt.-% of the melt blown fibre and/or melt blown web based on the melt blown fiber and from 70 wt.-% to 97 wt.-% of the fibrous substrate material, based on the total weight of the composite material. More preferably, the composite material comprises from 5 wt.-% to 30 wt.-% of the melt blown fibre and/or melt blown web based on the melt blown fiber and from 70 wt.-% to 95 wt.-% of the fibrous substrate material, based on the total weight of the composite material. Most preferably, the composite material comprises from 7 wt.-% to 30 wt.-% of the melt blown fibre and/or melt blown web based on the melt blown fiber and from 70 wt.-% to 93 wt.-% of the fibrous substrate material, based on the total weight of the composite material. For example, the composite material comprises from 7 wt.-% to 20 wt.-% of the melt blown fibre and/or melt blown web based on the melt blown fiber and from 80 wt.-% to 93 wt.-% of the fibrous substrate material, based on the total weight of the composite material.

The composite material as defined in the instant invention may be prepared by a process, comprising the steps of

(a) providing melt blown fiber prepared from the propylene copolymer as defined above,

(b) providing at least one fibrous substrate material as defined above,

(c) contacting a stream of the at least one fibrous substrate material of step (b) and a stream of the melt blown fiber of step (a) to form a composite material.

The steps of providing melt blown fiber, providing the at least one fibrous substrate material and contacting a stream of the at least one fibrous substrate material and a stream of the melt blown fiber may be undertaken by all the techniques well known to the man skilled in the art. For example, the composite material of the present invention may be prepared by a process as described as coform technique in WO 2011/034523 A1, US 4,100,324; US 5,350,624; US 5,508,102 and US patent application Nos. 2003/0200991 and 2007/0049153. The subject-matters of these documents relating to the coform technique are hereby incorporated by reference in its entirety.

The composite material of the present invention may be utilized in a wide variety of articles. For example, the composite material of the present invention may be incorporated in articles such as absorbent products and/or filtration media.

Examples of absorbent products comprise hygiene articles selected from the group consisting of diapers, incontinence articles and feminine hygiene articles, such as sanitary napkins; and/or tissue articles such as wipes selected from the group consisting of paper towels, bath tissue, facial tissue, wipes for babies and/or adults, cleaning wipes, polishing wipes, cosmetic wipes and car care wipes.

Tissue articles such as wipes may be provided in dry form or pre-moistened form. If the tissue article is a pre-moistened article, it may comprise a liquid such as for cleaning, disinfecting, sanitizing etc. Preferably, the liquid comprises water or oil in water emulsion. Preferably, the liquid comprises water in an amount of at least 80 wt.-%, more preferably at least 85 wt.-%, even more preferably at least 90 wt.-% and most preferably at least 95 wt.-%, based on the total weight of the liquid.

In one preferred embodiment of the present invention, the tissue article may comprise the liquid in an amount from 10 wt.-% to 2000 wt.-%, more preferably from 50 wt.-% to 1500 wt.-%, even more preferably from 100 wt.-% to 1000 wt.-% and most preferably from 200 wt.-% to 750 wt.-%, based on the total weight of the tissue article.

If the article is a filtration medium, the article is preferably a vacuum cleaner bag and/or a filter.

The absorbent products, i.e. hygiene articles and/or tissue articles, and/or filtration media may be provided in a wide variety of shapes. For example, the absorbent products and/or filtration media may be in a circular, oval, rectangular or irregularly shape.

The articles of the present invention may comprise in addition to the inventive composite material a spunbonded fabric known in the art.

In particular, it is appreciated that the inventive article has a maximum tensile strength in machine direction (MD) of at least 10 N measured according to ISO 527-3 on rectangular specimens cut from the article having 50 mm width and 140 mm length at a temperature of +23 ± 2 °C and a testing speed of 100 mm/min.

For example, the article has a maximum tensile strength in machine direction (MD) of between 10 N and 50 N, preferably between 15 N and 45 N and most preferably between 20 N and 40 N, measured according to ISO 527-3 on rectangular specimens cut from the article having 50 mm width and 140 mm length at a temperature of+23 ± 2 °C and a testing speed of 100 mm/min.

Additionally or alternatively, the inventive article has a maximum tensile strength in cross direction (CD) of at least 10 N measured according to ISO 527-3 on rectangular specimens cut from the article having 50 mm width and 140 mm length at a temperature of +23 ± 2 °C and a testing speed of 100 mm/min.

For example, the inventive article has a maximum tensile strength in cross direction (CD) of between 10 N and 40 N, preferably between 15 N and 35 N and most preferably between 15 N and 30 N measured according to ISO 527-3 on rectangular specimens cut from the article having 50 mm width and 140 mm length at a temperature of +23 ± 2 °C and a testing speed of 100 mm/min.

In this regard, it can be gathered that the propylene copolymer as defined above can also be used for improving the adhesion between a melt blown web and a fibrous substrate material in a composite material/article, especially compared to known composite materials/articles comprising a propylene homopolymer. Thus, it is appreciated that in a further aspect of the present invention the use of the propylene copolymer as defined above for improving the adhesion between a melt blown web and a fibrous substrate material in a composite material/article expressed by the maximum tensile strength oriented in machine direction (MD) is provided, wherein the improvement is defined by Formula (II)

$$(MD\text{-}RPP) \, / \, (MD\text{-}HPP) \geq 1.1 \qquad (II)$$

wherein

(MD-RPP) is the maximum tensile strength oriented in machine direction (MD) [N] of the propylene copolymer, and

(MD-HPP) is the maximum tensile strength in machine direction (MD) [N] of a propylene homopolymer having a melt flow rate $MFR_2$ (230 °C) measured according to ISO 1133 of 1100 g/10min to 1300 g/10min and a melting temperature ($T_m$) measured according to ISO 11357-3 of 155 °C to 160 °C

In view of the goods results achieved for the composite material/article with respect to the mechanical properties, in particular adhesion properties as expressed by the tensile strengths, by utilizing the propylene copolymer, a further aspect of the present invention refers to the use of the propylene copolymer as defined above for the preparation of a composite material as defined above or an article as defined above.

Furthermore, it should be noted that the composite material/article comprising such propylene copolymer can be prepared in a melt blowing process by using improved processing parameters. In particular, it is appreciated that the air volume required during the melt blowing process can be significantly reduced without losing the required properties of the melt-blown web and the articles obtained therefrom.

Thus, in a further aspect of the present invention the use of a propylene copolymer for reducing the hot air volume during a melt blowing process is provided, wherein the reduction is defined by Formula (I)

$$\text{(AV-RPP) / (AV-HPP)} \leq 0.9 \qquad \text{(I)}$$

wherein

(AV-RPP) is the hot air volume [$m^3$/h] of the propylene copolymer, and

(AV-HPP) is the hot air volume [$m^3$/h] of a propylene homopolymer having a melt flow rate $MFR_2$ (230 °C) measured according to ISO 1133 of 1100 g/10min to 1300 g/10min and a melting temperature ($T_m$) measured according to ISO 11357-3 of 155 °C to 160 °C.

In one preferred embodiment of the present invention, the reduction of the hot air volume by using the propylene copolymer [(AV-RPP) / (AV-HPP)] is more than 0.8 and more preferably more than 0.7, wherein (AV-RPP) is the hot air volume [$m^3$/h] of the propylene copolymer, and(AV-HPP) is the hot air volume [$m^3$/h] of a propylene homopolymer having a melt flow rate $MFR_2$ (230 °C) measured according to ISO 1133 of 1100 g/10min to 1300 g/10min and a melting temperature ($T_m$) measured according to ISO 11357-3 of 155 °C to 160 °C.

For example, the reduction of the hot air volume by using the propylene copolymer [(AV-RPP) / (AV-HPP)] is between 0.5 and 0.9, more preferably between 0.5 and 0.9 and most preferably between 0.6 and 0.9, wherein (AV-RPP) is the hot air volume [$m^3$/h] of the propylene copolymer, and(AV-HPP) is the hot air volume [$m^3$/h] of a propylene homopolymer having a melt flow rate $MFR_2$ (230 °C) measured according to ISO 1133 of 1100 g/10min to 1300 g/10min and a melting temperature ($T_m$) measured according to ISO 11357-3 of 155 °C to 160 °C.

The present invention will now be described in further detail by the examples provided below.

## 1. Definitions/Measuring Methods

The following definitions of terms and determination methods apply for the above general description of the invention as well as to the below examples unless otherwise defined.

## Quantification of isotacticity in polypropylene by $^{13}$C NMR spectroscopy

The isotacticity is determined by quantitative $^{13}$C nuclear magnetic resonance (NMR) spectroscopy after basic assignment as e.g. in: V. Busico and R. Cipullo, Progress in Polymer Science, 2001, 26, 443-533. Experimental parameters are adjusted to ensure measurement of quantitative spectra for this specific task as e.g in: S. Berger and S. Braun, 200 and More NMR Experiments: A Practical Course, 2004, Wiley-VCH, Weinheim. Quantities are calculated using simple corrected ratios of the signal integrals of representative sites in a manner known in the art. The isotacticity is determined at the pentad level i.e. mmmm fraction of the pentad distribution.

## Quantification of microstructure by NMR spectroscopy

Quantitative nuclear-magnetic resonance (NMR) spectroscopy was used to quantify the isotacticity, regio-regularity and comonomer content of the polymers.

Quantitative $^{13}$C{$^1$H} NMR spectra were recorded in the solution-state using a Bruker Advance III 400 NMR spectrometer operating at 400.15 and 100.62 MHz for $^1$H and $^{13}$C respectively. All spectra were recorded using a $^{13}$C optimised 10 mm extended temperature probehead at 125°C using nitrogen gas for all pneumatics.

For propylene homopolymers approximately 200 mg of material was dissolved in *1,2*-tetrachloroethane-$d_2$ (TCE-$d_2$). To ensure a homogenous solution, after initial sample preparation in a heat block, the NMR tube was further heated in a rotatary oven for at least 1 hour. Upon insertion into the magnet the tube was spun at 10 Hz. This setup was chosen primarily for the high resolution needed for tacticity distribution quantification (Busico, V., Cipullo, R., Prog. Polym. Sci. 26 (2001) 443; Busico, V.; Cipullo, R., Monaco, G., Vacatello, M., Segre, A.L., Macromoleucles 30 (1997) 6251). Standard single-pulse excitation was employed utilising the NOE and bi-level WALTZ 16 decoupling scheme (Zhou, Z., Kuemmerle, R., Qiu, X., Redwine, D., Cong, R., Taha, A., Baugh, D. Winniford, B., J. Mag. Reson. 187 (2007) 225; Busico, V., Carbonniere, P., Cipullo, R., Pellecchia, R., Severn, J., Talarico, G., Macromol. Rapid Commun. 2007, 28, 11289). A total of 8192 (8k) transients were acquired per spectra

For ethylene-propylene copolymers approximately 200 mg of material was dissolved in 3 ml of $1,2$-tetrachloroethane-$d_2$ (TCE-$d_2$) along with chromium-(III)-acetylacetonate (Cr(acac)$_3$) resulting in a 65 mM solution of relaxation agent in solvent (Singh, G., Kothari, A., Gupta, V., Polymer Testing 28 5 (2009), 475). To ensure a homogenous solution, after initial sample preparation in a heat block, the NMR tube was further heated in a rotatary oven for at least 1 hour. Upon insertion into the magnet the tube was spun at 10 Hz. This setup was chosen primarily for the high resolution and quantitatively needed for accurate ethylene content quantification. Standard single-pulse excitation was employed without NOE, using an optimised tip angle, 1 s recycle delay and a bi-level WALTZ 16 decoupling scheme(Zhou, Z., Kuemmerle, R., Qiu, X., Redwine, D., Cong, R., Taha, A., Baugh, D. Winniford, B., J. Mag. Reson. 187 (2007) 225; Busico, V., Carbonniere, P., Cipullo, R., Pellecchia, R., Severn, J., Talarico, G., Macromol. Rapid Commun. 2007, 28, 11289). A total of 6144 (6k) transients were acquired per spectra.

Quantitative $^{13}C\{^1H\}$ NMR spectra were processed, integrated and relevant quantitative properties determined from the integrals using proprietary computer programs.

For ethylene-propylene copolymers all chemical shifts were indirectly referenced to the central methylene group of the ethylene block (EEE) at 30.00 ppm using the chemical shift of the solvent. This approach allowed comparable referencing even when this structural unit was not present.

For propylene homopolymers all chemical shifts are internally referenced to the methyl isotactic pentad (mmmm) at 21.85 ppm.

Characteristic signals corresponding to regio defects (Resconi, L., Cavallo, L., Fait, A., Piemontesi, F., Chem. Rev. 2000, 100, 1253;; Wang, W-J., Zhu, S., Macromolecules 33 (2000), 1157; Cheng, H. N., Macromolecules 17 (1984), 1950) or comonomer were observed.

The tacticity distribution was quantified through integration of the methyl region between 23.6-19.7 ppm correcting for any sites not related to the stereo sequences of interest(Busico, V., Cipullo, R., Prog. Polym. Sci. 26 (2001) 443; Busico, V., Cipullo, R., Monaco, G., Vacatello, M., Segre, A.L., Macromoleucles 30 (1997) 6251).

Specifically the influence of regio defects and comonomer on the quantification of the tacticity distribution was corrected for by subtraction of representative regio defect and comonomer integrals from the specific integral regions of the stereo sequences.

The isotacticity was determined at the pentad level and reported as the percentage of isotactic pentad (mmmm) sequences with respect to all pentad sequences:

$$[mmmm] \% = 100 * ( mmmm / sum of all pentads )$$

The presence of 2,1 erythro regio defects (<2,1> regiodefects) was indicated by the presence of the two methyl sites at 17.7 and 17.2 ppm and confirmed by other characteristic sites. Characteristic signals corresponding to other types of regio defects were not observed (Resconi, L., Cavallo, L., Fait, A., Piemontesi, F., Chem. Rev. 2000, 100, 1253).

The amount of 2,1 erythro regio defects was quantified using the average integral of the two characteristic methyl sites at 17.7 and 17.2 ppm:

$$P_{21e} = ( I_{e6} + I_{e8} ) / 2$$

The amount of 1,2 primary inserted propene was quantified based on the methyl region with correction undertaken for sites included in this region not related to primary insertion and for primary insertion sites excluded from this region:

$$P_{12} = I_{CH3} + P_{12e}$$

The total amount of propene was quantified as the sum of primary inserted propene and all other present regio defects:

$$\mathbf{P_{total} = P_{12} + P_{21e}}$$

The mole percent of 2,1 erythro regio defects was quantified with respect to all propene:

$$[21e] \text{ mol\%} = 100 * ( P_{21e} / P_{total} )$$

For copolymers characteristic signals corresponding to the incorporation of ethylene were observed (Cheng, H. N., Macromolecules 17 (1984), 1950).

With regio defects also observed (Resconi, L., Cavallo, L., Fait, A., Piemontesi, F., Chem. Rev. 2000, 100, 1253; Wang, W-J., Zhu, S., Macromolecules 33 (2000), 1157; Cheng, H. N., Macromolecules 17 (1984), 1950) correction for the influence of such defects on the comonomer content was required.

The mole fraction of ethylene in the polymer was quantified using the method of Wang et. al. (Wang, W-J., Zhu, S., Macromolecules 33 (2000), 1157) through integration of multiple signals across the whole spectral region of a $^{13}C\{^{1}H\}$ spectra acquired using defined conditions. This method was chosen for its accuracy, robust nature and ability to account for the presence of regio-defects when needed. Integral regions were slightly adjusted to increase applicability to a wider range of comonomer contents.

The mole percent comonomer incorporation in the polymer was calculated from the mole fraction according to:

$$E \text{ [mol\%]} = 100 * fE$$

The weight percent comonomer incorporation in the polymer was calculated from the mole fraction according to:

$$E \text{ [wt\%]} = 100 * ( fE * 28.05 ) / ( (fE * 28.05) + ((1\text{-}fE) * 42.08) )$$

The comonomer sequence distribution at the triad level was determined using the method of Kakugo et al. (Kakugo, M., Naito, Y., Mizunuma, K., Miyatake, T. Macromolecules 15 (1982) 1150) through integration of multiple signals across the whole spectral region of a $^{13}C\{^{1}H\}$ spectra acquired using defined conditions. This method was chosen for its robust nature. Integral regions were slightly adjusted to increase applicability to a wider range of comonomer contents.

The mole percent of a given comonomer triad sequence in the polymer was calculated from the mole fraction determined by the method of Kakugo et at. (Kakugo, M., Naito, Y., Mizunuma, K., Miyatake, T. Macromolecules 15 (1982) 1150) according to:

$$XXX \text{ [mol\%]} = 100 * fXXX$$

The mole fraction comonomer incorporation in the polymer, as determined from the comonomer sequence distribution at the triad level, were calculated from the triad distribution using known necessary relationships (Randall, J. Macromol. Sci., Rev. Macromol. Chem. Phys. 1989, C29, 201):

$$fXEX = fEEE + fPEE + fPEP$$

$$fXPX = fPPP + fEPP + fEPE$$

where PEE and EPP represents the sum of the reversible sequences PEE/EEP and EPP/PPE respectively.

The randomness of the comonomer distribution was quantified as the relative amount of isolated ethylene sequences as compared to all incorporated ethylene. The randomness was calculated from the triad sequence distribution using the relationship:

$$R(E) [\%] = 100 * ( fPEP / fXEX )$$

**Randomness**

In the FTIR measurements, films of 250 -mm thickness were compression moulded at 225 °C and investigated on a Perkin-Elmer System 2000 FTIR instrument. The ethylene peak area (760-700 $cm^{-1}$) was used as a measure of total ethylene content. The absorption band for the structure -P-E-P- (one ethylene unit between propylene units), occurs at 733 $cm^{-1}$. This band characterizes the random ethylene content. For longer ethylene sequences (more than two units), an absorption band occurs at 720 $cm^{-1}$. Generally, a shoulder corresponding to longer ethylene runs is observed for the random copolymers. The calibration for total ethylene content based on the area and random ethylene (PEP) content based on peak height at 733 $cm^{-1}$ was made by $^{13}$CNMR. (Thermochimica Acta, 66 (1990) 53-68 ).

$$\text{Randomness} = \text{random ethylene (-P-E-P-) content / the total ethylene content x } 100\%.$$

**Number average molecular weight ($M_n$), weight average molecular weight ($M_w$) and molecular weight distribution (MWD)** are determined by size exclusion chromatography (SEC) using Waters Alliance GPCV 2000 instrument with online viscometer. The oven temperature is 140 °C. Trichlorobenzene is used as a solvent (ISO 16014).
**$MFR_2$ (230 °C)** is measured according to ISO 1133 (230 °C, 2.16 kg load).

**Quantification of comonomer content by FTIR spectroscopy**

Quantitative infrared (IR) spectroscopy was used to quantify the amount of comonomer. Calibration was achieved by correlation to comonomer contents determined by quantitative nuclear magnetic resonance (NMR) spectroscopy.
The calibration procedure based on results obtained from quantitative $^{13}$C-NMR spectroscopy was undertaken in the conventional manner well documented in the literature. The amount of comonomer (N) was determined as weight percent (wt%) via:

$$N = k1 (A / R) + k2$$

where A is the maximum absorbance defined of the comonomer band, R the maximum absorbance defined as peak height of the reference peak and with k1 and k2 the linear constants obtained by calibration.
For poly(ethylene-co-propene-co-butene) (EPB) systems quantification was achieved via two characteristic absorbance bands:

- 1-butene content via 760 $cm^{-1}$ (linear baseline correction between 750 to 810 $cm^{-1}$)
- ethylene content via 720 $cm^{-1}$ or 730 $cm^{-1}$ (linear baseline correction between 710 to 750 $cm^{-1}$)

The band used for ethane content quantification is selected depending if the ethylene content is random (730 $cm^{-1}$) or block-like (720 $cm^{-1}$). The absorbance at 4324 $cm^{-1}$ was used as a reference band.
The amount of comonomer (N) as determined as mole percent (mol%) was obtained by conversion from units of weight percent (wt%) through use of the molecular weight (Mw) of the comonomer:

$$NA[mol\%] = (NA[wt\%] / MwA ) / ( (NA[wt\%] / MwA ) + ( NB[wt\%] / MwB ) + ( ( 100[wt\%] - NA[wt\%] - NB[wt\%] ) / MwC ) )$$

Solid-state infrared spectra were recorded using a FTIR spectrometer on compression molded thin (100-800 $\mu$m) films at a resolution of 4 $cm^{-1}$ and analysed in transmission mode. **Tensile Modulus of the polymers** is evaluated according to ISO 527-1 (cross head speed = 1 mm/min; 23 °C) using injection molded specimens as described in EN ISO 1873-2 (dog bone shape, 4 mm thickness).

**Melting temperature Tm**

Melting temperature (peak temperature) Tm was measured by DSC according to ISO 11357-1using the peak temperature in the second heat in a heat - cool - heat cycle with a rate of 10 K/min between ambient temperature and 210°C.

**The melt enthalpy (Hm)** [heat of fusion] was measured by the DSC method according to ISO 11357-3.

**Crystallinity**

The crystallinity of the polymers were calculated from the melting enthalpy Hm in a standard differential scanning calorimetry (DSC) experment according to ISO 3146 running at a heating rate of 10 K/min and assuming a melting enthalpy of 209 J/g for a completely crystalline propylene homopolymer (see e.g. the following reference: Markus Gahleitner, Pirjo Jääskeläinen, Ewa Ratajski, Christian Paulik, Jens Reussner, Johannes Wolfschwenger & Wolfgang Neiss 1, Propylene-Ethylene Random Copolymers: Comonomer Effects on Crystallinity and Application Properties, J.Appl.Polym.Sci. 95 (2005) 1073-81 ).

**The xylene solubles (XCS**, **wt.-%)**: Content of Xylene solubles (XCS) is determined at 23 °C according ISO 6427.

**Grammage of the web**

The basis weight (grammage) of the webs in $g/m^2$ was determined in accordance with ISO 536:1995.

**Tensile properties of the web**

The tensile strength of the webs was determined in alignment to ISO 527-3 on rectangular specimens cut from the web having 50 mm width and 140 mm length with an effective clamping length of 100 mm using a Zwick 005 tensile tester. The specimens were cut in machine (MD) and cross direction (CD) The test temperature was +23 $\pm$ 2 °C and a testing speed of 100 mm/min was used up to the breakage point, extension being measured by distance of clamps based on initial distance of clamps. 10 samples were tested from which an average curve of force vs. extension was calculated. From these average curves the tensile strength was determined as the maximum force reached and the elongations at maximum force was determined

**2. Preparation of the Examples**

**2.1 Polymer samples**

**Inventive example (IE):**

A random propylene copolymer polymerized using a commercial 4th generation Ziegler-Natta catalyst having an $MFR_2$ of 20 g/10min and an ethylene content as determined by FTIR of 3.3 wt.-% was used as a starting material for visbreaking. The visbreaking was performed in a co-rotating twin-screw extruder at 200-230°C using an appropriate amount of (tert.butylperoxy)-2,5-dimethylhexane (Trigonox 101, distributed by Akzo Nobel, Netherlands) to achieve an $MFR_2$ of 1200 g/10min. The resulting polymer is characterized by a melting point of 150 °C, a tensile modulus of 1150 MPa as determined on injection-molded specimens,

**Comparative example (CE):**

The commercial propylene homopolymer Borflow™ HL512FB (Borealis) having a $MFR_2$ of 1200 g/10min produced in a visbreaking process was used. This polymer is characterized by a melting point of 158°C and a tensile modulus of 1515 MPa as determined on injection-moulded specimens.

**2.2 Melt Blown webs and testing**

The materials have been converted into melt blown webs on a 250 mm wide Reicofil melt blown pilot line using a die with holes of 0.4 mm diameter and 35 holes per inch. The melt temperature was set at 285 °C and the air temperature at 280 °C. The throughput of the line was 66 kg/h/m and the distance from die to collector was fixed at 150 mm, 300 mm and 500 mm, respectively. For each setting the hot air volume has been maximized and webs produced having a basis weight of 50 $g/m^2$.

Table 1 summarizes data regarding air volume and mechanical properties with respect to inventive example IE and comparative example CE for the respective distances from die to collector.

Table 1:

| Sample | DCD | Air volume | Max. tensile strength MD | Max. tensile strength CD | Elongation MD | Elongation CD |
|---|---|---|---|---|---|---|
| | [mm] | [m$^3$/h] | [N] | [N] | [%] | [%] |
| CE | 150 | 220 | 22.9 | 7.2 | 1.2 | 2 |
| CE | 300 | 330 | 26.1 | 16.8 | 18 | 34 |
| CE | 500 | 410 | 16.1 | 14.7 | 22 | 40 |
| IE | 150 | 190 | 19.5 | 8.1 | 5.1 | 18.2 |
| IE | 300 | 230 | 27.9 | 12.6 | 24 | 11.5 |
| IE | 500 | 280 | 17.1 | 16.8 | 20 | 19 |

From the results obtained, it can be gathered that the mechanical properties for the melt blown webs prepared from inventive example IE are comparable to those prepared from comparative example CE.

**2**.**3 Composite preparation and testing**

Inventive example IE as well as comparative example CE has been deposed on different paper substrates by using a 33 g/m$^2$. Toilet paper as well as a 40 g/m$^2$ industrial cleaning paper were used as paper substrate. The melt temperature was set at 285°C and the air temperature at 280 °C. The throughput of the line was 66 kg/h/m and the distance from die to collector was fixed at 150 mm, while keeping a melt blown weight of 50 g/m$^2$.
Table 2 summarizes data regarding air volume and mechanical properties with respect to inventive example IE and comparative example CE deposed on different paper substrates.

Table 2:

| Sample | Substrate | Air volume | Max. tensile strength MD | Max. tensile strength CD | Elongation MD | Elongation CD |
|---|---|---|---|---|---|---|
| | | [m$^3$/h] | [N] | [N] | [%] | [%] |
| CE | Toilet paper | 220 | 21.9 | 18.2 | 0.9 | 0.9 |
| CE | Cleaning paper | 220 | 10.2 | 9.7* | 0.8 | 0.8* |
| IE | Toilet paper | 190 | 24.5 | 20.9 | 1.7 | 1.2 |
| IE | Cleaning paper | 190 | 32.7 | 12.9* | 2 | 1,2* |
| *: measured with 50 mm initial length | | | | | | |

As can be gathered from the tensile strengths measured for the composite material comprising a paper substrate and comparative example CE, this polymer shows hardly any adhesion to the fibrous substrate material. In contrast thereto, the composite material comprising inventive example IE and paper substrates as fibrous substrate material shows increased tensile strengths. Thus, the composite material prepared from inventive example IE shows improved adhesion compared to a composite material prepared from a propylene homopolymer (as shown for comparative example CE). Furthermore, it can be gathered that the use of inventive example IE for preparing such a composite requires significantly lower hot air volumes compared to the use of comparative example CE.

**Claims**

**1.** Composite material comprising

(a) melt blown fiber, wherein

(i) said melt blown fiber comprise at least 85 wt.-% of a propylene copolymer having a comonomer content of 0.5 to 7.0 wt.-%, the comonomer is ethylene and/or at least one $C_4$ to $C_{20}$ $\alpha$-olefin,

(ii) optionally said melt blown fibers and/or said propylene copolymer has/have a melt flow rate $MFR_2$ (230 °C) measured according to ISO 1133 of at least 200 g/10min, and

(b) at least one fibrous substrate material, wherein

the composite material comprises 1 to 30 wt.-% of the melt blown fiber and 70 to 99 wt.-% of the fibrous substrate material, based on the total weight of the composite material.

2. The composite material according to claim 1, wherein the propylene copolymer and/or melt blown fiber has/have a melting temperature of at least 130 °C.

3. The composite material according to claims 1 or 2, wherein the melt blown fiber and/or propylene copolymer has/have a melt flow rate $MFR_2$ (230 °C) measured according to ISO 1133 in the range of 200 g/10min and 3000 g/10min.

4. The composite material according to any one of the preceding claims, wherein the propylene copolymer has <2,1> regiodefects of not more than 0.4 mol.-% determined by $^{13}$C-spectroscopy.

5. The composite material according to any one of the preceding claims, wherein the pentad isotacticity of the propylene copolymer is higher than 95 mol.-% determined with $^{13}$C NMR spectroscopy.

6. The composite material according to any one of the preceding claims, wherein the propylene copolymer has been visbroken with a visbreaking ratio [final $MFR_2$ (230 °C) / initial $MFR_2$ (230 °C)] of 5 to 100, wherein

"initial $MFR_2$ (230 °C)" is the $MFR_2$ (230 °C) of the propylene copolymer before visbreaking and

"final $MFR_2$ (230 °C)" is the $MFR_2$ (230 °C) of the propylene copolymer after visbreaking.

7. The composite material according to any one of the preceding claims, wherein the comonomer of the propylene copolymer is ethylene.

8. The composite material according to any one of the preceding claims, wherein the fibrous substrate material is based on a material selected from the group consisting of wood pulp, rayon, lyocell, cotton, wool, silk, jute, linen, hemp, flax, polyester, nylons, polyethylene, polyethylene terephthalate, polyacrylate, salts of polyacrylate, polycarbonate and mixtures thereof.

9. The composite material according to any one of the preceding claims, wherein the melt blown fiber are in form of a melt blown web.

10. The composite material according to claim 9, wherein the melt blown web has a maximum tensile strength

(a) in machine direction (MD) of at least 10 N measured according to ISO 527-3 on rectangular specimens cut from the web having 50 mm width and 140 mm length at a temperature of +23 $\pm$ 2 °C and a testing speed of 100 mm/min and/or

(b) in cross direction (CD) of at least 5 N measured according to ISO 527-3 on rectangular specimens cut from the web having 50 mm width and 140 mm length at a temperature of +23 $\pm$ 2 °C and a testing speed of 100 mm/min.

11. Article comprising a composite material according to any one of the preceding claims 1 to 10, said article is selected from filtration media and/or absorbent products such as hygiene articles and/or tissue articles.

12. Article according to claim 11, wherein the article has a maximum tensile strength

(a) in machine direction (MD) of at least 10 N measured according to ISO 527-3 on rectangular specimens cut from the article having 50 mm width and 140 mm length at a temperature of +23 $\pm$ 2 °C and a testing speed of 100 mm/min and/or

(b) in cross direction (CD) of at least 10 N measured according to ISO 527-3 on rectangular specimens cut from the article having 50 mm width and 140 mm length at a temperature of +23 $\pm$ 2 °C and a testing speed of 100 mm/min.

13. Process for the preparation of a composite material according to any one of the claims 1 to 10 comprising the steps of

(a) providing a melt blown fiber prepared from the propylene copolymer as defined in any one of claims 1 to 7,
(b) providing at least one fibrous substrate material as defined in claims 1 or 8,
(c) contacting a stream of the at least one fibrous substrate material of step (b) and a stream of the melt blown fiber of step (a) to form a composite material.

14. Use of a propylene copolymer as defined in any one of claims 1 to 7 for the preparation of a composite material as defined in any one of the claims 1 to 10 or an article as defined in claims 1 or 12.

15. Use of a propylene copolymer as defined in any one of claims 1 to 7, in preparing a composite material as defined in any one of claims I to 10 or an article as defined in any one of claims 11 to 12, for reducing the hot air volume during a melt blowing process wherein the reduction is defined by Formula (I)

$$(AV\text{-}RPP) \,/\, (AV\text{-}HPP) \leq 0.9 \qquad\qquad (I)$$

wherein
(AV-RPP) is the hot air volume [$m^3/h$] of the propylene copolymer, and (AV-HPP) is the hot air volume [$m^3/h$] of a propylene homopolymer having a melt flow rate $MFR_2$ (230 °C) measured according to ISO 1133 of 1100 g/10min to 1300 g/10min and a melting temperature ($T_m$) measured according to ISO 11357-3 of 155 °C to 160 °C.

## Patentansprüche

1. Verbundmaterial, umfassend

(a) Schmelz-geblasene Fasern, wobei

(i) die Schmelz-geblasenen Fasern mindestens 85 Gew.-% von einem Propylen-Copolymer mit einem Comonomer-Gehalt von 0,5 bis 7,0 Gew.-% umfassen, wobei das Comonomer Ethylen und/oder mindestens ein $C_4$ bis $C_{20}$ $\alpha$-Olefin ist,
(ii) gegebenenfalls die Schmelz-geblasenen Fasern und/oder das Propylen-Copolymer eine Schmelze-Fließ-Rate $MFR_2$ (230°C), gemessen gemäß ISO 1133, von mindestens 200 g/10 min aufweist/aufweisen, und

(b) mindestens ein faserartiges Substrat-Material, wobei
das Verbundmaterial 1 bis 30 Gew.-% der Schmelz-geblasenen Fasern und 70 bis 99 Gew.-% des faserartigen Substrat-Materials, basierend auf dem Gesamt-Gewicht des Verbundmaterials, umfasst.

2. Verbundmaterial nach Anspruch 1, wobei das Propylen-Copolymer und/oder Schmelz-geblasene Fasern eine Schmelz-Temperatur von mindestens 130°C aufweist/aufweisen.

3. Verbundmaterial nach Ansprüchen 1 oder 2, wobei die Schmelz-geblasenen Fasern und/oder Propylen-Copolymer eine Schmelze-Fließ-Rate $MFR_2$ (230°C), gemessen gemäß ISO 1133, in dem Bereich von 200 g/10 min und 3000 g/10 min aufweist/aufweisen.

4. Verbundmaterial nach einem der vorangehenden Ansprüche, wobei das Propylen-Copolymer <2,1> Regiodefekte von nicht mehr als 0,4 Mol-%, bestimmt durch $^{13}$C-Spektroskopie, aufweist.

5. Verbundmaterial nach einem der vorangehenden Ansprüche, wobei die Pentaden-Isotaktizität des Propylen-Copolymers höher als 95 Mol-%, bestimmt mit $^{13}$C NMR-Spektroskopie, ist.

6. Verbundmaterial nach einem der vorangehenden Ansprüche, wobei das Propylen-Copolymer mit einem Visbreaking-Verhältnis [End-$MFR_2$ (230°C) / Anfangs-$MFR_2$ (230°C)] von 5 bis 100 Visbreaking unterzogen wurde, wobei "Anfangs-$MFR_2$ (230°C)" die $MFR_2$ (230°C) des Propylen-Copolymers vor dem Visbreaking ist und "End-$MFR_2$ (230°C)" die $MFR_2$ (230°C) des Propylen-Copolymers nach dem Visbreaking ist.

7. Verbundmaterial nach einem der vorangehenden Ansprüche, wobei das Comonomer des Propylen-Copolymers Ethylen ist.

8. Verbundmaterial nach einem der vorangehenden Ansprüche, wobei das faserartige Substrat-Material auf einem Material basiert, ausgewählt aus der Gruppe, bestehend aus Holzzellstoff, Reyon, Lyocell, Baumwolle, Wolle, Seide, Jute, Leinen, Hanf, Flachs, Polyester, Nylons, Polyethylen, Polyethylenterephthalat, Polyacrylat, Salzen von Polyacrylat, Polycarbonat und Gemischen davon.

9. Verbundmaterial nach einem der vorangehenden Ansprüche, wobei die Schmelz-geblasenen Fasern in Form eines Schmelz-geblasenen Flächengebildes vorliegen.

10. Verbundmaterial nach Anspruch 9, wobei das Schmelz-geblasene Flächengebilde eine maximale Zugfestigkeit

(a) in Maschinen-Richtung (MD) von mindestens 10 N, gemessen gemäß ISO 527-3 an rechtwinkligen Probestücken, geschnitten aus dem Flächengebilde mit 50 mm Breite und 140 mm Länge bei einer Temperatur von +23 ± 2°C und einer Test-Geschwindigkeit von 100 mm/min und/oder
(b) in Quer-Richtung (CD) von mindestens 5 N, gemessen gemäß ISO 527-3, an rechtwinkligen Probestücken, geschnitten aus dem Flächengebilde mit 50 mm Breite und 140 mm Länge bei einer Temperatur von +23 ± 2°C und einer Test-Geschwindigkeit von 100 mm/min, aufweist.

11. Gegenstand, umfassend ein Verbundmaterial nach einem der vorangehenden Ansprüche 1 bis 10, wobei der Gegenstand aus Filtrations-Medien und/oder Absorptionsmittel-Produkten, wie Hygiene-Artikeln und/oder Tissue-Artikeln, ausgewählt ist.

12. Gegenstand nach Anspruch 11, wobei der Gegenstand eine maximale Zugfestigkeit

(a) in Maschinen-Richtung (MD) von mindestens 10 N, gemessen gemäß ISO 527-3, an rechtwinkligen Probestücken, geschnitten aus dem Gegenstand mit 50 mm Breite und 140 mm Länge bei einer Temperatur von +23 ± 2°C und einer Test-Geschwindigkeit von 100 mm/min und/oder
(b) in Quer-Richtung (CD) von mindestens 10 N, gemessen gemäß ISO 527-3, an rechtwinkligen Probestücken, geschnitten aus dem Gegenstand mit 50 mm Breite und 140 mm Länge bei einer Temperatur von +23 ± 2°C und einer Test-Geschwindigkeit von 100 mm/min, aufweist.

13. Verfahren zur Herstellung eines Verbundmaterials nach einem der Ansprüche 1 bis 10, umfassend die Schritte von

(a) Bereitstellen einer Schmelz-geblasenen Faser, hergestellt aus dem Propylen-Copolymer, wie in einem der Ansprüche 1 bis 7 definiert,
(b) Bereitstellen mindestens eines faserartigen Substrat-Materials, wie in Ansprüchen 1 oder 8 definiert,
(c) In-Kontakt-Bringen eines Stroms des mindestens einen faserartigen Substrat-Materials von Schritt (b) und eines Stroms der Schmelz-geblasenen Faser von Schritt (a), um ein Verbundmaterial zu bilden.

14. Verwendung eines Propylen-Copolymers, wie in einem der Ansprüche 1 bis 7 definiert, zur Herstellung eines Verbundmaterials, wie in einem der Ansprüche 1 bis 10 definiert, oder eines Gegenstands, wie in Ansprüchen 11 oder 12 definiert.

15. Verwendung eines Propylen-Copolymers, wie in einem der Ansprüche 1 bis 7 definiert, zur Herstellung eines Verbundmaterials, wie in einem der Ansprüche 1 bis 10 definiert, oder eines Gegenstands, wie in einem der Ansprüche 11 bis 12 definiert, zur Verminderung des Heiß-Luft-Volumens während eines Schmelz-Blas-Verfahrens, wobei die Verminderung definiert wird durch die Formel (I)

$$(AV\text{--}RPP) \; / \; (AV\text{--}HPP) \; \leq \; 0,9 \qquad (I)$$

wobei
(AV-RPP) das Heiß-Luft-Volumen [$m^3$/h] des Propylen-Copolymers ist, und
(AV-HPP) das Heiß-Luft-Volumen [$m^3$/h] von einem Propylen-Homopolymer mit einer Schmelze-Fließ-Rate $MFR_2$ (230°C), gemessen gemäß ISO 1133, von 1100 g/10 min bis 1300 g/10 min und einer Schmelz-Temperatur ($T_m$),

gemessen gemäß ISO 11357-3, von 155°C bis 160°C ist.

**Revendications**

1. Matériau composite comprenant

   (a) des fibres soufflées à l'état fondu,

      (i) lesdites fibres soufflées à l'état fondu comprenant au moins 85 % en poids d'un copolymère de propylène présentant une teneur en comonomère de 0,5 à 7,0 % en poids, le comonomère étant l'éthylène et/ou au moins une a-oléfine en $C_4$ à $C_{20}$,
      (ii) éventuellement lesdites fibres soufflées à l'état fondu et/ou ledit copolymère de propylène présentant un indice de fluage $MFR_2$ (230°C), mesuré conformément à la norme ISO 1133, d'au moins 200 g/10 min, et

   (b) au moins un matériau substrat fibreux,
   lequel matériau composite comprend 1 à 30 % en poids des fibres soufflées à l'état fondu et 70 à 99 % en poids du matériau de substrat fibreux, par rapport au poids total du matériau composite.

2. Matériau composite selon la revendication 1, dans lequel le copolymère de propylène et/ou les fibres soufflées à l'état fondu présente(nt) un point de fusion d'au moins 130°C.

3. Matériau composite selon la revendication 1 ou 2, dans lequel les fibres soufflées à l'état fondu et/ou le copolymère de propylène présente(nt) un indice de fluage $MFR_2$ (230°C), mesuré conformément à la norme ISO 1133, situé dans la plage allant de 200 g/10 min à 3000 g/10 min.

4. Matériau composite selon l'une quelconque des revendications précédentes, dans lequel le copolymère de propylène présente au plus 0,4 % en moles de régiodéfauts <2,1>, déterminés par spectroscopie [13]C.

5. Matériau composite selon l'une quelconque des revendications précédentes, dans lequel l'isotacticité de pentade du copolymère de propylène, déterminée par spectroscopie RMN-$^{13}$C, est supérieure à 95 % en moles.

6. Matériau composite selon l'une quelconque des revendications précédentes, dans lequel le copolymère de propylène a été viscoréduit à un rapport de viscoréduction [$MFR_2$ final (230°C) / $MFR_2$ initial (230°C)] de 5 à 100, où "$MFR_2$ initial (230°C)" est le $MFR_2$ (230°C) du copolymère de propylène avant viscoréduction, et "$MFR_2$ final (230°C)" est le $MFR_2$ (230°C) du copolymère de propylène après viscoréduction.

7. Matériau composite selon l'une quelconque des revendications précédentes, dans lequel le comonomère du copolymère de propylène est l'éthylène.

8. Matériau composite selon l'une quelconque des revendications précédentes, dans lequel le matériau de substrat fibreux est à base d'un matériau choisi dans l'ensemble constitué par la cellulose, la rayonne, le lyocel, le coton, la laine, la soie, le jute, la toile de lin, le chanvre, le lin, le polyester, les nylons, le polyéthylène, le poly(téréphtalate d'éthylène), le polyacrylate, les sels de polyacrylate, le polycarbonate, et leurs mélanges.

9. Matériau composite selon l'une quelconque des revendications précédentes, dans lequel les fibres soufflées à l'état fondu sont sous la forme d'une toile soufflée à l'état fondu.

10. Matériau composite selon la revendication 9, dans lequel la toile soufflée à l'état fondu présente une résistance à la traction maximale

   (a) dans le sens machine (MD) d'au moins 10 N, telle que mesurée conformément à la norme ISO 527-3 sur des échantillons rectangulaires découpés dans la toile ayant une largeur de 50 mm et une longueur de 140 mm à une température de +23 $\pm$ 2°C et à une vitesse de test de 100 mm/min, et/ou
   (b) dans le sens travers (CD) d'au moins 5 N, telle que mesurée conformément à la norme ISO 527-3 sur des échantillons rectangulaires découpés dans la toile ayant une largeur de 50 mm et une longueur de 140 mm à une température de +23 $\pm$ 2°C et à une vitesse de test de 100 mm/min.

**11.** Article comprenant un matériau composite selon l'une quelconque des revendications 1 à 10, ledit article étant choisi parmi les milieux de filtration et/ou les produits absorbants tels que les articles d'hygiène et/ou les articles en tissu.

**12.** Article selon la revendication 11, lequel article présente une résistance à la traction maximale

(a) dans le sens machine (MD) d'au moins 10 N, telle que mesurée conformément à la norme ISO 527-3 sur des échantillons rectangulaires découpés dans l'article ayant une largeur de 50 mm et une longueur de 140 mm à une température de +23 $\pm$ 2°C et à une vitesse de test de 100 mm/min, et/ou
(b) dans le sens travers (CD) d'au moins 10 N, telle que mesurée conformément à la norme ISO 527-3 sur des échantillons rectangulaires découpés dans l'article ayant une largeur de 50 mm et une longueur de 140 mm à une température de +23 $\pm$ 2°C et à une vitesse de test de 100 mm/min.

**13.** Procédé pour la préparation d'un matériau composite selon l'une quelconque des revendications 1 à 10, comprenant les étapes

(a) de fourniture de fibres soufflées à l'état fondu préparées à partir du copolymère de propylène comme défini dans l'une quelconque des revendications 1 à 7,
(b) de fourniture d'au moins un matériau de substrat fibreux comme défini dans la revendication 1 ou 8,
(c) de mise en contact d'un courant de l'au moins un matériau de substrat fibreux de l'étape (b) avec un courant des fibres soufflées à l'état fondu de l'étape (a) pour former un matériau composite.

**14.** Utilisation d'un copolymère de propylène tel que défini dans l'une quelconque des revendications 1 à 7 pour la préparation d'un matériau composite tel que défini dans l'une quelconque des revendications 1 à 10 ou d'un article tel que défini dans la revendication 11 ou 12.

**15.** Utilisation d'un copolymère de propylène tel que défini dans l'une quelconque des revendications 1 à 7 dans la préparation d'un matériau composite tel que défini dans l'une quelconque des revendications 1 à 10 ou d'un article tel que défini dans la revendication 11 ou 12, pour réduire le volume d'air chaud durant un procédé de soufflage à l'état fondu, dans laquelle la réduction est définie par la formule (I)

$$(AV-RPP)/(AV-HPP) \leq 0,9 \qquad\qquad (I)$$

dans laquelle
(AV-RPP) est le volume d'air chaud [m$^3$/h] du copolymère de propylène, et
(AV-HPP) est le volume d'air chaud [m$^3$/h] d'un homopolymère de propylène présentant un indice de fluage MFR$_2$ (230°C), mesuré conformément à la norme ISO 1133, de 1100 g/10 min à 1300 g/10 min, et un point de fusion ($T_m$), mesuré conformément à la norme ISO 11357-3, de 155°C à 160°C.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2011092092 A
- WO 2011077277 A
- WO 2007024447 A
- US 5234879 A
- WO 9219653 A
- WO 9219658 A
- WO 9933843 A
- EP 0887379 A1

- WO 9212182 A
- WO 2011034523 A1
- US 4100324 A
- US 5350624 A
- US 5508102 A
- US 20030200991 A
- US 20070049153 A

**Non-patent literature cited in the description**

- **P. VILLE et al.** *Polymer,* 2001, vol. 42, 1953-1967
- **V. BUSICO ; R. CIPULLO.** *Progress in Polymer Science,* 2001, vol. 26, 443-533
- **S. BERGER ; S. BRAUN.** More NMR Experiments: A Practical Course. Wiley-VCH, 2004, 200
- **BUSICO, V. ; CIPULLO, R.** *Prog. Polym. Sci.,* 2001, vol. 26, 443
- **BUSICO, V. ; CIPULLO, R. ; MONACO, G. ; VACATELLO, M. ; SEGRE, A.L.** *Macromoleucles,* 1997, vol. 30, 6251
- **ZHOU, Z. ; KUEMMERLE, R. ; QIU, X. ; REDWINE, D. ; CONG, R. ; TAHA, A. ; BAUGH, D. ; WINNIFORD, B.** *J. Mag. Reson,* 2007, vol. 187, 225
- **BUSICO, V. ; CARBONNIERE, P. ; CIPULLO, R. ; PELLECCHIA, R. ; SEVERN, J. ; TALARICO, G.** *Macromol. Rapid Commun.,* 2007, vol. 28, 11289
- **SINGH, G. ; KOTHARI, A. ; GUPTA, V.** *Polymer Testing,* 2009, vol. 28 (5), 475
- **ZHOU, Z. ; KUEMMERLE, R. ; QIU, X. ; REDWINE, D ; CONG, R. ; TAHA, A. ; BAUGH, D ; WINNIFORD, B.** *J. Mag. Reson.,* 2007, vol. 187, 225

- **RESCONI, L. ; CAVALLO, L. ; FAIT, A. ; PIEMONTESI, F.** *Chem. Rev.,* 2000, vol. 100, 1253
- **WANG, W-J. ; ZHU, S.** *Macromolecules,* 2000, vol. 33, 1157
- **CHENG, H. N.** *Macromolecules,* 1984, vol. 17, 1950
- **RESCONI, L. ; CAVALLO, L. ; FAIT, A. ; PIEMONTESI, F.** *Chem. Rev,* 2000, vol. 100, 1253
- **KAKUGO, M. ; NAITO, Y. ; MIZUNUMA, K. ; MIYATAKE, T.** *Macromolecules,* 1982, vol. 15, 1150
- **RANDALL.** *J. Macromol. Sci., Rev. Macromol. Chem. Phys.,* 1989, vol. C29, 201
- *Thermochimica Acta,* 1990, vol. 66, 53-68
- **MARKUS GAHLEITNER ; PIRJO JÄÄSKELÄINEN ; EWA RATAJSKI ; CHRISTIAN PAULIK ; JENS REUSSNER ; JOHANNES WOLFSCHWENGER ; WOLFGANG NEISS 1.** Propylene-Ethylene Random Copolymers: Comonomer Effects on Crystallinity and Application Properties. *J.Appl.Polym.Sci.,* 2005, vol. 95, 1073-81